# EUROPEAN PATENT APPLICATION

(11) **EP 2 236 159 A2**
(43) Date of publication of application: **06.10.2010**
(21) Application number: 10158233.6
(22) Date of filing: 29.03.2010
(51) Int. Cl.: A61K 47/30, A61K 9/20, A61K 31/437

(54) **Modified release dimebolin compositions**

(30) Priority: 31.03.2009 TR 200902500
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Turkyilmaz, Ali, 34398, Istanbul (TR)
(74) Representative: Sevinç, Erkan

(57) **Abstract**

According to the present invention a modified release formulation comprising dimebolin HCl and an alginate salt and/or complex salt of alginic acid, together with one or more pharmaceutically acceptable excipients is provided.

## Description

### Technical Aspect

The present invention is related to a modified release formulation comprising dimebolin HCl and an alginate salt and/or complex salt of alginic acid, together with one or more pharmaceutically acceptable excipients.

### Background of the Invention

Dimebolin is an antiallergic agent and NMDA antagonist. Its chemical name is 2,8-dimethyl-5-(2-(6-methyl-3-pyridyl)ethyl)-2,3,4,5-tetrahydro-IH-pyrido[4,3-b]indole and its chemical structure is shown in the Formula I.

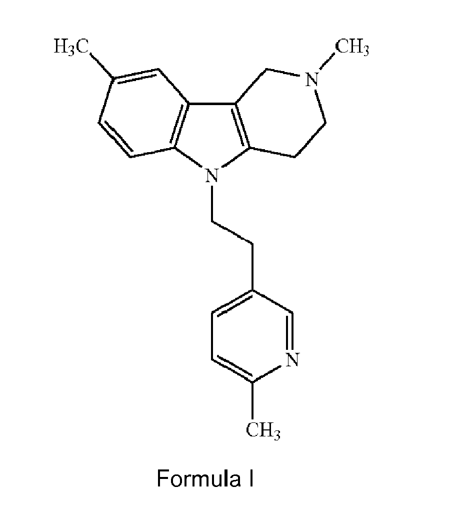

Dimebolin has been used as an antiallergic agent (Inventor's Certificate No. 1138164, IP Class A61K 31/47,5, C07 D 209/52, published on Feb. 7, 1985) in Russia for over 20 years. It has hereto been administered orally in the form of tablets containing a dose from 2.5 mg to 20 mg. The usual average prescription is 1 tablet in 3 times a day.

As described in U.S. Patent No. 6,187,785 and 7,071,206, hydrogenated pyrido[4,3-b]indole derivatives, such as dimebolin, have NMDA antagonist properties, which make them useful for treating neurodegenerative diseases, such as Alzheimer's disease. As described in WO 2005/055951, hydrogenated pyrido[4,3-b]indole derivatives, such as dimebolin, are useful as human or veterinary geroprotectors. They work by delaying the onset and/or development of an age-associated or related manifestation and/or pathology or condition, including disturbance in skin-hair integument, vision disturbance and weight loss. U.S. Provisional Patent Application No. 60/723,403, filed October 4, 2006, and U.S. Patent Application No. 11/543,529, filed October 4, 2006, disclose hydrogenated pyrido[4,3- b] indole derivatives, such as dimebolin, as neuroprotectors for use in treating and/or preventing and/or slowing the progression or onset and/or development of Huntington's disease. See also RU application filed January 25, 2006 with an English language title of "Agent for Treatment of Schizophrenia Based on Hydrogenated Pyrido [4,3 -b] indoles (Variations), a Pharmacological Agent Based on it, and a Method of Using it."

It is desirable in the treatment of a number of diseases, to provide the active pharmaceutical ingredient in a modified release form. Desirably the modified release provides a generally uniform and constant rate of release over a modified period of time which achieves desired plasma level of the active ingredient without the need for frequent administration of the medicament.

Because of several different reasons it is difficult to develop modified release formulations of highly soluble pharmaceuticals although there are many modified release formulations formulated with hydrophilic and/or hydrophobic polymers as well as alginate salt and/or complex salt of alginic acid. First of all, modified release formulations of highly soluble medicaments can be prone to "dose dumping" in which the release of the active ingredient is delayed but once the release begins the medicament is released very fast. It is also hard to achieve the desired dissolution profiles in other words the control of the release rate is difficult. Therefore, fluctuation of the active ingredient concentration in the plasma may occur which may lead to toxicity. Also, diurnal variation of the active ingredient in plasma is also possible.

Accordingly, a need rises for modified release formulations of soluble medicaments such as dimebolin HCl, which overcomes one or more of the above described handicaps and which further provide the advantageous property of allowing the active medicament to be administered less frequently, e.g. once-a-day or twice-a-day, while achieving comparable plasma levels to immediate release form.

### Description of the invention

According to the present invention a modified release formulation comprising dimebolin HCl and an alginate salt and/or complex salt of alginic acid, together with one or more pharmaceutically acceptable excipients is provided.

In one embodiment the amount of dimebolin HCl is present in 10 to 60% by weight of total formulation, particularly 25 to 35%.

The present invention relates to the modified release formulations comprising dimebolin HCl thereof, is preferably in an amount of 1 mg to 420mg. The modified release formulation is to be administered once-a-day or twice-a-day.

The formulations of the invention comprise one or more excipients. Such excipients include diluents, fillers, disintegrants, binders, lubricants, glidants, and preservatives.

Suitable diluents and fillers may include but not limited to lactose, microcrystalline cellulose, starch, mannitol, glucose, and the like and mixtures thereof; preferably starch and microcrystalline cellulose.

Suitable disintegrants may include but not limited to microcrystalline cellulose, sodium starch glycollate, croscarmellose sodium, crospovidone, starch and the like and mixtures thereof.

Suitable binders may include but not limited to povidone, sucrose, polyethylene glycol and the like and mixtures thereof, preferably povidone.

Suitable lubricants may include but not limited to magnesium strearate, calcium stearate, sodium stearyl fumarate, stearic acid and the like and mixtures thereof, preferably magnesium strearate and stearic acid.

Suitable glidants may include but not limited to colloidal silicon dioxide, talc, aluminium silicate and the like and mixtures thereof, preferably colloidal silicon dioxide.

Suitable preservatives may include but not limited to methyl paraben, propyl paraben, sodium benzoate, citric acid and benzoic acid and the like and mixtures thereof, preferably citric acid.

These modified release pharmaceutical formulations are administrated by oral, parenteral, intramuscular and topical route. The modified release pharmaceutical formulations of the invention include tablet, capsule, sachet, microcapsules, minitablet, multilayer and multicoated tablet, pellet, injectable preparat, suspension, syrup, gel, cream or ointment which can be formulated in accordance with methods that are standard in the art

One of the main objects of the present invention is the amount of alginate salt and/or complex salt of alginic acid, preferably sodium alginate and/or sodium-calcium alginate is preferably selected to provide the formulation releases of maximum 30% of the total amount of dimebolin HCl in 2 hours and 40-65% in 6 hours and at least 85% between a period of 16 to 24 hours.

The term complex salt of alginic acid is sodium-calcium alginate. The amount of sodium-calcium alginate is 0,2 to 10% by weight of total formulation particularly 0.5 to 5%.

The term alginate salt includes substance such as calcium alginate, potassium alginate, sodium alginate, propylene glycol alginate, and derivatives and mixtures thereof. The alginate salt is preferably sodium alginate.

The alginate salt, preferably sodium alginate is conveniently present in 0.5 to 20% by weight of total formulation, particularly 5 to 10%.

The formulation contains one or more pharmaceutically acceptable excipients are selected from the group comprising (a) 4 to 60 % by weight of starch, (b) 1 to 10% by weight of povidone, (c) 5 to 20% by weight of citric acid, (d) 0.5 to 10% by weight of stearic acid and (e) 0.1 to 5% by weight of magnesium stearate.

The formulations of the present invention may be prepared by conventional technology well known to those skilled in the art such as wet granulation, direct compression, dry granulation and the like. Thus, for instance, dimebolin HCl, the alginate salt and/or complex salt of alginic acid and other excipients are mixed together to form the modified release formulations. Preferably dimebolin HCl, the alginate salt and/or complex salt of alginic acid and other excipients are mixed together then the mixture is compressed to form tablets or it is filled into capsules.

The mixing process is preferably performed by mixing the components, wet granulating the mixed components; the mixed components, drying the mixture, milling the dried mixture, blending the mixture with a lubricant(s) and compressing the blended mixture to form tablets or filling the blended mixture into capsules.

A preferred process for preparing the formulations of the invention comprises the following steps:
(a) mixing dimebolin HCl and the alginate salt and/or complex salt of alginic acid and other excipients;
(b) wet granulating the mixing components;
(c) drying the mixture;
(d) milling the dried mixture;
(e) blending the mixture with a lubricant; and
(f) compressing the blended mixture to form tablets, and optionally coating said tablets.

The dosage forms may be coated with one or more coatings as is well known in the art such as, for example, shellac, zein, hydroxyproply cellulose, hydroxypropyl methylcellulose, ethly cellulose, polymethacrylates, polyvinyl acetate phthalate, cellulose acetate phthalate, triacetin, dibutyl sebacate, polyethylene glycol, titanium dioxide, and the like and mixtures thereof.

The modified release pharmaceutical formulation is preferably enteric coated.

Therefore, further aspects of the present invention concern the use of pharmaceutical formulations comprising dimebolin HCl, together with one or more pharmaceutically acceptable excipients for the manufacture of a medicament for the treatment of Alzheimer disease, neuropathic pain, chronic visceral pain, chronic inflammatory pain, headache, antihistaminic disease in a warm- blooded animal.

The formulations which is in the form of a tablet comprising:
(a) a core comprising the dimebolin HCl and pharmaceutically acceptable excipients,
(b) a coating layer comprising the a hydrophilic and/or hydrophobic polymer and an alginate salt and/or complex salt of alginic acid.

## Claims

1. A modified release formulation comprising dimebolin HCl and an alginate salt and/or complex salt of alginic acid, together with one or more pharmaceutically acceptable excipients.

2. The modified release formulation according to claim 1 wherein dimebolin HCl is present in 10 to 60% by weight of total formulation.

3. The modified release formulation according to claim 1 wherein the maximum 30% of total amount of dimebolin HCl is released in 2 hours and 40-65% in 6 hours and at least 85% between a period of 16 to 24 hours.

4. The modified release formulation according to claim 1 wherein the complex salt of alginic acid is sodium-calcium alginate.

5. The modified release formulation according to claim 4 wherein the amount of sodium-calcium alginate is 0,2 to 10% by weight of total formulation.

6. The modified release formulation according to claim 1 wherein the alginate salt is selected from the group comprising calcium alginate, potassium alginate, sodium alginate, propylene glycol alginate, and derivatives and mixtures thereof.

7. The modified release formulation according to claim 1 and 6 wherein the alginate salt is sodium alginate.

8. The modified release formulation according to claim 7 wherein the amount of sodium alginate is 0.5 to 20% by weight of total formulation.

9. The modified release formulation according to any preceding claim wherein the one or more pharmaceutically acceptable excipients are selected from the group comprising (a) 4 to 60 % by weight of starch, (b) 1 to 10% by weight of povidone, (c) 5 to 20% by weight of citric acid, (d) 0.5 to 10% by weight of stearic acid and (e) 0.1 to 5% by weight of magnesium stearate.

10. The modified release formulations according to anyone of claims 1- 9 further comprising the coating layer.

11. The modified release formulation according to claim 10 wherein the coating layer is preferably enteric coating.

12. The modified release formulations according to any preceding claim, in the form of a tablet comprising:
(c) a core comprising the dimebolin HCl,
(d) a coating layer comprising the a hydrophilic and/or hydrophobic polymer and an alginate salt and/or complex salt of alginic acid.

13. The use according to any preceding claim, wherein the modified release composition is to be administrated by oral, parenteral, intramuscular or topical route.

14. The use according to any preceding claim, wherein the modified release composition is in the form of a tablet, capsule, sachet, microcapsule, minitablet, multilayer and multicoated tablet, pellet, injectable preparat, suspension, syrup, gel, cream or ointment.

15. The process for preparing modified release formulations according to any preceding claim which comprises
(a) mixing dimebolin HCl and the alginate salt and/or complex salt of alginic acid and other excipients;
(b) wet granulating the mixed components;
(c) drying the mixture;
(d) milling the dried mixture;
(e) blending the mixture with a lubricant; and
(f) compressing the blended mixture to form tablets, and optionally coating said tablets.
